(19) Europäisches Patentamt
European Patent Office
Office européen des brevets

(11) Veröffentlichungsnummer: **0 368 160 A1**

(12) **EUROPÄISCHE PATENTANMELDUNG**

(21) Anmeldenummer: **89120367.1**

(22) Anmeldetag: **03.11.89**

(51) Int. Cl.5: **C07D 207/38, C07D 213/64, C07D 213/70, C07D 213/14, C07D 237/16, C07D 237/18, C07D 239/36, C07D 239/40, A61K 31/44**

(30) Priorität: **08.11.88 DE 3837809**

(43) Veröffentlichungstag der Anmeldung: **16.05.90 Patentblatt 90/20**

(84) Benannte Vertragsstaaten: **AT BE CH DE ES FR GB GR IT LI LU NL SE**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Baumgarth, Manfred, Dr.**
**Sachsenstrasse 53**
**D-6100 Darmstadt(DE)**
Erfinder: **Gericke, Rolf, Dr.**
**Mozartstrasse 19**
**D-6104 Seeheim(DE)**
Erfinder: **Bergmann, Rolf, Dr.**
**Birkenhag 36**
**D-6101 Reichelsheim(DE)**
Erfinder: **De Peyer, Jacques, Dr.**
**Zinsgutstrasse 14**
**D-6102 Pfungstadt(DE)**
Erfinder: **Lues, Ingeborg, Dr.**
**Paul-Wagner Strasse 13**
**D-6100 Darmstadt(DE)**

(54) **Tetralinderivate.**

(57) Neue Tetralinderivate der Formel I

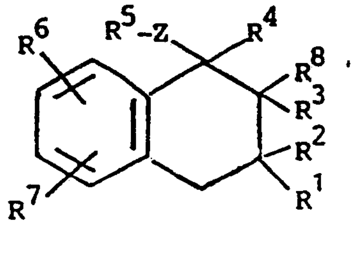

I

worin $R^1$ bis $R^8$ und Z die in Patentanspruch 1 angegebenen Bedeutungen haben, sowie ihre Salze zeigen Wirkungen auf das cardiovaskuläre System.

EP 0 368 160 A1

## Tetralinderivate

Die Erfindung betrifft Tetralinderivate der Formel I

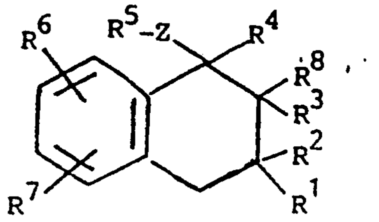

I

worin
$R^1$, $R^2$ und $R^8$ jeweils H oder A,
$R^1$ und $R^2$ zusammen auch Alkylen mit 3-6 C-Atomen,
$R^3$ H, OH, OA oder OAc,
$R^4$ H,
$R^3$ und $R^4$ zusammen auch eine Bindung,
$R^5$ einen unsubstituierten oder ein- oder zweifach durch A, F, Cl, Br, J, OH, OA, OAc, SH, $NO_2$, $NH_2$, AcNH, HOOC und/oder AOOC substituierten Pyridyl-, Pyridazinyl-, Pyrimidinyl- Pyrazinyl-, Oxo-dihydro-pyridyl-, Oxo-dihydro-pyri dazinyl-, Oxo-dihydro-pyrimidinyl-, Oxo-dihydro-pyrazinyl- oder Oxo-dihydro-pyrrolylrest,
$R^6$ und $R^7$ jeweils H, A, HO, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitroalkyl, Cyanalkyl, A-C(=NOH) oder A-C(=$NH_2$),
Z O, S, NH oder eine Bindung,
A Alkyl mit 1-6 C-Atomen,
-alkyl Alkylen mit 1-6 C-Atomen und
Ac Alkanoyl mit 1-8 C-Atomen oder Aroyl mit 7-11 C-Atomen
bedeuten,
sowie deren Salze.

Ähnliche Verbindungen sind bekannt aus der EP-A-168619.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z. B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z. B. in EP-A-76075, EP-A-168619, EP-A-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z. B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls $R^1$ und $R^2$ zusammen Alkylen bedeuten, so ist die Alkylengruppe vorzugsweise unverzweigt, im einzelnen bevorzugt $-(CH_2)_n-$, wobei n 3, 4, 5 oder 6 bedeutet.

Die Gruppe "-alkyl" steht vorzugsweise für $-CH_2-$ oder $-CH_2CH_2-$.

Ac ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m- oder p-Toluyl, 1- oder 2-Naphthoyl.

$R^1$ und $R^2$ sind vorzugsweise jeweils Alkyl, insbesonderė jeweils Methyl oder Ethyl, bevorzugt jeweils Methyl.

$R^3$ und $R^4$ sind bevorzugt zusammen eine Bindung. Falls $R^4$ H bedeutet, ist $R^3$ bevorzugt OH, O-CHO oder $O-COCH_3$.

Falls Z eine Bindung bedeutet, ist $R^5$ bevorzugt unsubstituiertes 2-Oxo-1,2-dihydro-1-pyridyl(1H-2-Pyridon-1-yl), 3-Hydroxy-6-oxo-1,6-dihydro-pyridazin-1-yl oder 2-Oxo-4-hydroxy-1,2-dihydro-1-pyridyl, ferner bevorzugt unsubstituiertes 2-Oxo-1,2-dihydro-pyrazin-1-yl, 6-Oxo-1,6-dihydro-pyridazin-1-yl, 2-Oxo-1,2-dihydro-pyrimidin-1-yl, 6-Oxo-1,6-dihydro-pyrimidin-1-yl, 2-Oxo-2,3- oder -2,5-dihydro-pyrrol-1-yl oder 2-Thioxo-1,2-dihydro-pyridin-1-yl. Falls $R^5$ einen substituierten Pyridon- bzw. Thiopyridonring bedeutet, so ist dieser Ring vorzugsweise einfach in 3-, 4- oder 5-Stellung oder zweifach in 3- und 5-Stellung substituiert. Besonders bevorzugte Substituenten sind OH, $NO_2$ und $NH_2$, ferner AOOC, OA, Cl, Br und $NHCOCH_3$, besonders bevorzugte substituierte Reste $R^5$ im einzelnen 4-, ferner 3-, 5- und 6-Hydroxy-, 3-, 4-, 5- oder 6-Methoxy-, 3-, 4-, 5- oder 6-Acetoxy-, 3-, 5- oder 6-Chlor-, 3- oder 5-Nitro-, 3- oder 5-Amino-, 3- oder 5-Carboxy-, 3- oder 5-Methoxycarbonyl-, 3- oder 5-Ethoxycarbonyl-, 3- oder 5-Acetamido-, 3,5-Dichlor-, 3,5-Dibrom-, 3-Chlor-5-nitro-, 3-Nitro-5-chlor-, 3-Brom-5-nitro-, 3-Nitro-5-brom-, 3,5-Dinitro-, 3-Chlor-5-amino-, 3-Amino-5-chlor-, 3-Brom-5-amino-, 3-Amino-5-brom-, 3-Chlor-5-acetamido-, 3-Acetamido-5-chlor-, 3-Brom-5-acetamido- und 3-Acetamido-5-brom-2-oxo-1,2-dihydro-1-pyridyl bzw. -2-thioxo-1,2-dihydro-1-pyridyl, 4- oder 5-Hydroxy-6-oxo-1,6-dihydro-pyridazin-1-yl, 3-, 4- oder 5-Methoxy-6-oxo-1,6-dihydro-pyridazin-1-yl, 3-, 4- oder 5-Ethoxycarbonyl-6-oxo-1,6-dihydro-pyrimidin-1-yl, 2-oder 4-Hydroxy-6-oxo-1,6-dihydro-pyrimidin-1-yl.

Falls Z O, S oder NH bedeutet, ist $R^5$ bevorzugt 6-Hydroxy-3-pyridazinyl (= 1,6-Dihydro-6-oxo-3-pyridazinyl) oder 2-Hydroxy-4-pyridyl (= 1,2-Dihydro-2-oxo-4-pyridyl), ferner bevorzugt unsubstituiertes 2-, 3- oder 4-Pyridyl, 2-, 4- oder 5-Pyrimidinyl, 3-, 4- oder 5- Pyridazinyl oder Pyrazinyl, Hydroxy-pyridyl wie 3-, 4-, 5- oder 6-Hydroxy-2-pyridyl, 2-, 4- oder 5-Hydroxy-3-pyridyl, 3-Hydroxy-4-pyridyl, 2-Hydroxy-5-pyridyl; Hydroxy-pyridazinyl wie 4-oder 5-Hydroxy-3-pyridazinyl, 3-, 5- oder 6-Hydroxy-4-pyridazinyl; Hydroxy-pyrimidinyl wie 4- oder 5-Hydroxy-2-pyrimidinyl, 2-, 5- oder 6-Hydroxy-4-pyrimidinyl, 2- oder 4-Hydroxy-5-pyrimidinyl; Hydroxy-pyrazinyl wie 3-, 5-oder 6-Hydroxy-2-pyrazinyl; Dihydro-alkyl-oxo-pyridyl wie 1,2-Dihydro-1-methyl-2-oxo-3-, -4-, -5- oder -6-pyridyl, 1,2-Dihydro-1-ethyl-2-oxo-3-, -4-, -5- oder -6-pyridyl; Dihydro-alkyl-oxo-pyridazinyl wie 1,6-Dihydro-1-methyl-6-oxo-3-, -4- oder -5-pyridazinyl, 1,6-Dihydro-1-ethyl-6-oxo-3-, -4- oder -5-pyridazinyl; Alkoxy-pyridyl wie 3-, 4-, 5- oder 6-Methoxy-2-pyridyl, 2-, 4- oder 5-Methoxy-3-pyridyl, 2- oder 3-Methoxy-4-pyridyl, 2-Methoxy-5-pyridyl, 2- oder 3-Ethoxy-4-pyridyl; Alkoxy-pyridazinyl wie 4-, 5- oder 6-Methoxy-3-pyridazinyl, 4-, 5- oder 6-Ethoxypyridazinyl, 3-, 5- oder 6-Methoxy-4-pyridazinyl, 3-, 5- oder 6-Ethoxy-4-pyridazinyl; Alkoxy-pyrimidinyl wie 4- oder 5-Methoxy-2-pyrimidinyl, 2-, 5- oder 6-Methoxy-4-pyrimidinyl, 2- oder 4-Methoxy-5-pyrimidinyl; Alkoxy-pyrazinyl wie 3-, 5- oder 6-Methoxy-2-pyrazinyl; Amino-pyridyl wie 3-, 4-, 5- oder 6-Aminopyridyl, 2-, 4- oder 5-Amino-3-pyridyl, 2- oder 3-Amino-4-pyridyl, 2-Amino-5-pyridyl; Amino-pyridazinyl wie 4-, 5- oder 6-Amino-3-pyridazinyl, 3-, 5- oder 6-Amino-4-pyridazinyl; Amino-pyrimidinyl wie 4- oder 5-Amino-2-pyrimidinyl, 2-, 5- oder 6-Amino-4-pyrimidinyl, 2- oder 4-Amino-5-pyrimidinyl; Amino-pyrazinyl wie 3-, 5- oder 6-Amino-2-pyrazinyl; Mercapto-pyridyl wie 3-, 4-, 5- oder 6-Mercapto-2-pyridyl, 2-, 4-oder 5-Mercapto-3-pyridyl, 2- (= 1,2-Dihydro-2-thioxo-4-pyridyl) oder 3-Mercapto-4-pyridyl, 2-Mercapto-5-pyridyl; Mercapto-pyridazinyl wie 4-, 5- oder 6-Mercapto-3-pyridazinyl (= 1,6-Dihydro-6-thioxo-3-pyridazinyl), 3-, 5- oder 6-Mercapto-4-pyridazinyl; Mercapto-pyrimidinyl wie 4- oder 5-Mercapto-2-pyrimidinyl, 2-, 5- oder 6-Mercapto-4-pyrimidinyl, 2- oder 4-Mercapto-5-pyrimidinyl; Mercapto-pyrazinyl wie 3-, 5- oder 6-Mercapto-2-pyrazinyl.

Diejenigen Reste $R^5$, die eine einem Ring-N-Atom benachbarte Hydroxy- oder Mercaptogruppe enthalten, können auch in der tautomeren Lactam- oder Thio-lactam-form vorliegen, wie oben in Einzelfällen angegeben.

In $R^6$ und $R^7$ bedeuten vorzugsweise:

A: Methyl, ferner Ethyl;

AO: Methoxy, ferner Ethoxy;

ACO: Acetyl, ferner Propionyl;

ACS: Thioacetyl, ferner Thiopropionyl;

AOOC: Methoxycarbonyl, ferner Ethoxycarbonyl;

AO-CS: Methoxy-thiocarbonyl, ferner Ethoxy-thiocarbonyl;

ACOO: Acetoxy, ferner Propionoxy;

ACSO: Thio(no)acetoxy, ferner Thio(no)propionoxy;
Hydroxyalkyl: Hydroxymethyl oder 1- oder 2-Hydroxyethyl;
Mercaptoalkyl: Mercaptomethyl oder 1- oder 2-Mercaptoethyl;
NHA: Methylamino, ferner Ethylamino;
$NA_2$: Dimethylamino, ferner Diethylamino;
ASO: Methylsulfinyl, ferner Ethylsulfinyl;
$ASO_2$: Methylsulfonyl, ferner Ethylsulfonyl;
AO-SO: Methoxy-sulfinyl, ferner Ethoxy-sulfinyl;
AO-$SO_2$: Methoxy-sulfonyl, ferner Ethoxy-sulfonyl;
Ac-NH: Acetamido, ferner Formamido, Propionamido oder Benzamido;
AO-CO-NH: Methoxycarbonylamino, ferner Ethoxycarbonylamino;
HANSO: Methylaminosulfinyl, ferner Ethylaminosulfinyl
$A_2$NSO: Dimethylaminosulfinyl, ferner Diethylaminosulfinyl;
$HANSO_2$: Methylaminosulfonyl, ferner Ethylaminosulfonyl;
$A_2NSO_2$: Dimethylaminosulfonyl, ferner Diethylaminosulfonyl;
HANCO: N-Methylcarbamoyl, ferner N-Ethylcarbamoyl;
$A_2$NOC: N,N-Dimethylcarbamoyl, ferner N,N-Dimethylcarbamoyl;
HANCS: N-Methyl-thiocarbamoyl, ferner N-Ethyl-thiocarbamoyl;
$A_2$NCS: N,N-Dimethyl-thiocarbamoyl, ferner N,N-Diethyl-thiocarbamoyl;
ASONH: Methylsulfonylamino, ferner Ethylsulfinylamino;
$ASO_2$NH: Methylsulfonylamino, ferner Ethylsulfonylamino;
AOSONH: Methoxysulfinylamino, ferner Ethoxysulfinylamino;
$AOSO_2$NH: Methoxysulfonylamino, ferner Ethoxysulfonylamino;
ACO-alkyl: 2-Oxopropyl, 2-Oxobutyl, 3-Oxobutyl, 3-Oxopentyl;
Nitroalkyl: Nitromethyl, 1- oder 2-Nitroethyl;
Cyanalkyl: Cyanmethyl, 1- oder 2-Cyanethyl;
A-C(= NOH): 1-Oximinoethyl, ferner 1-Oximinopropyl;
A-C(= $NNH_2$): 1-Hydrazonoethyl, ferner 1-Hydrazonopropyl.

Die Reste $R^6$ und $R^7$ stehen vorzugsweise in 6- und 7-Stellung des Tetralinsystems. Sie können jedoch auch in 5- und 6-, 5- und 7-, 5- und 8-, 6- und 8- sowie 7- und 8-Stellung stehen.

Von den Resten $R^6$ und $R^7$ ist vorzugsweise der eine H, während der andere von H verschieden ist. Dieser andere Rest steht vorzugsweise in 6-Stellung, aber auch in 5-, 7- oder 8-Stellung, und ist vorzugsweise CN oder $NO_2$, ferner bevorzugt CHO, ACO (insbesondere Acetyl), AOOC (insbesondere Methoxycarbonyl oder Ethoxycarbonyl), ACOO (insbesondere Acetoxy), weiterhin bevorzugt F, Cl, Br, J, $CF_3$, $H_2$NCO, $H_2$NCS oder $NH_2$.

Der Rest $R^8$ ist vorzugsweise H, weiterhin bevorzugt Methyl oder Ethyl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen Verbindungen können durch die nachstehenden Formeln Ia bis Ii ausgedrückt werden, die der Formel I entsprechen und worin die nicht näher bezeichneten Reste die bei der Formel I angegebene Bedeutung haben, worin jedoch

in Ia $R^1$ und $R^2$ jeweils A bedeuten;

in Ib $R^1$ und $R^2$ jeweils $CH_3$ bedeuten;

in Ic $R^1$ und $R^2$ zusammen Alkylen mit 3-6 C-Atomen bedeuten;

in Id $R^5$-Z- 2-Oxo-1,2-dihydro-1-pyridyl, 2-Oxo-4-hydroxy-1,2-dihydro-1-pyridyl oder 3-Hydroxy-6-oxo-1,6-dihydro-1-pyridazinyl bedeutet;

in Ie $R^5$-Z- 2-Pyridyloxy, 2-Hydroxy-4-pyridyloxy oder 6-Hydroxy-3-pyridazinyloxy bedeutet;

In If $R^5$-Z- 2-Oxo-1,2-dihydro-1-pyridyl bedeutet;

in Ig $R^1$ und $R^2$ jeweils $CH_3$ oder zusammen Alkylen mit 3-6 C-Atomen,
$R^5$-Z- 2-Oxo-1,2-dihydro-1-pyridyl, 2-Oxo-4-hydroxy-1,2-dihydro-1-pyridyl oder 3-Hydroxy-6-oxo-1,6-dihydro-1-pyridazinyl und
$R^8$ H oder $CH_3$ bedeuten.

in Ih $R^1$ und $R^2$ jeweils $CH_3$,
$R^5$ 2-Oxo-1,2-dihydro-1-pyridyl, 2-Oxo-4-hydroxy-1,2-dihydro-1-pyridyl oder 3-Hydroxy-6-oxo-1,6-dihydro-1-pyridazinyl und
$R^8$ H oder $CH_3$ bedeuten;

in Ii $R^1$ und $R^2$ jeweils $CH_3$,
$R^5$ 2-Oxo-1,2-dihydro-1-pyridyl und

$R^8$ H oder $CH_3$ bedeuten.

Weiterhin sind bevorzugt Verbindungen der Formeln I' sowie Ia' bis Ii', die den Formeln I sowie Ia bis Ii entsprechen, worin jedoch jeweils zusätzlich $R^3$ OH, OCHO oder $OCOCH_3$ und $R^4$ H bedeuten.

Weiterhin sind bevorzugt Verbindungen der Formeln I'' sowie Ia'' bis Ii'', die den Formeln I sowie Ia bis Ii entsprechen, worin jedoch jeweils zusätzlich $R^3$ und $R^4$ zusammen eine Bindung bedeuten.

Ferner sind bevorzugt Verbindungen der Formeln I, I', I'', Ia bis Ii, Ia' bis Ii' sowie Ia'' bis Ii'', worin jeweils zusätzlich

(a) $R^6$ von H verschieden ist und
$R^7$ H bedeutet;

(b) $R^6$ von H verschieden ist und in 6-Stellung steht und
$R^7$ H bedeutet;

(c) $R^6$ $NO_2$, CN, CHO, ACO, HOOC, AOOC, ACOO, F, Cl, Br, J, $CF_3$, $H_2NCO$, $H_2NCS$ oder $NH_2$ und
$R^7$ H bedeutet;

(d) $R^6$ $NO_2$, CN, CHO, ACO, HOOC, AOOC, ACOO, F, Cl, Br, J, $CF_3$, $H_2NCO$, $H_2NCS$ oder $NH_2$ bedeutet und in 6-Stellung steht und
$R^7$ H bedeutet;

(e) $R^6$ $NO_2$, CN, CHO, $CH_3CO$, $CH_3OOC$,$C_2H_5OOC$ oder $CH_3COO$ und
$R^7$ H bedeutet;

(f) $R^6$ $NO_2$, CN, CHO, $CH_3CO$, $CH_3OOC$,$C_2H_5OOC$ oder $CH_3COO$ bedeutet und in 6-Stellung steht und
$R^7$ H bedeutet;

(g) $R^6$ $NO_2$ oder CN und
$R^7$ H bedeutet;

(h) $R^6$ $NO_2$ oder CN bedeutet und in 6-Stellung steht und
$R^7$ H bedeutet;

(i) $R^6$ CN und
$R^7$ H bedeutet;

(j) $R^6$ CN bedeutet und in 6-Stellung steht und
$R^7$ H bedeutet.

Insbesondere sind bevorzugt Verbindungen der Formeln I, I', I'', Ia bis Ii, Ia' bis Ii', Ia'' bis Ii'' sowie der übrigen vorstehend als bevorzugt gekennzeichneten Gruppen von Verbindungen, worin zusätzlich $R^8$ H bedeutet.

Im übrigen haben vor- und nachstehend die Reste $R^1$ bis $R^8$, A, "-alkyl" und Ac die bei Formel I angegebenen Bedeutungen, wenn nicht ausdrücklich etwas anderes angegeben ist.

Gegenstand der Erfindung ist ferner ein Verfahren zur Herstellung von Tetralinderivaten der Formel I gemäß Anspruch 1, dadurch gekennzeichnet, daß man ein Tetralin der Formel II

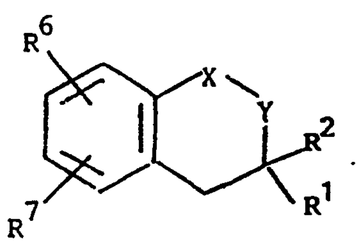

II

worin
X-Y

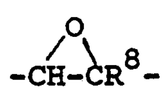

oder $-CHE-CR^3R^8-$ und
E Cl, Br, J oder eine reaktionsfähig veresterte OH-Gruppe bedeuten und
$R^1$, $R^2$, $R^3$, $R^6$, $R^7$ und $R^8$ die bei Formel I angegebenen Bedeutungen haben,
mit einer Verbindung der Formel III

$R^5$-ZH III

worin $R^5$ und Z die bei Formel I angegebenen Bedeutungen haben,
oder mit einem ihrer reaktionsfähigen Derivate umsetzt und/oder daß man eine Verbindung der Formel I, worin $R^3$ OH und $R^4$ H bedeutet, dehydratisiert und/oder daß man in einer Verbindung der Formel I einen oder mehrere der Reste $R^3$, $R^5$, $R^6$ und/oder $R^7$ in andere Reste $R^3$, $R^5$, $R^6$ und/oder $R^7$ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150 °. Dabei können, abhängig von den Reaktionsbedingungen und der Konstitution der Ausgangsstoffe, nebeneinander Verbindungen der Formel I entstehen, worin $R^3$ H und $R^4$ OH oder worin $R^3$ und $R^4$ zusammen eine Doppelbindung bedeuten. Ferner können bei Verwendung von Ausgangsstoffen der Formel III, die eine -CO-NH-Gruppe enthalten (z.B. 1H-2-Pyridon), mit dem Tetralinring über das N-Atom [z.B. 4-(2-Oxo-1,2-dihydro-pyridyl)-tetraline] oder über eine O-Brücke [z.B. 4-(2-Pyridyl-oxy)-tetraline] verbundene Verbindungen der Formel I nebeneinander entstehen.

Ausgangsstoffe der Formel II mit

X-Y = 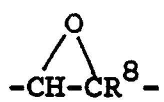 (3,4-Epoxy-tetraline)

sind bevorzugt.

Die Ausgangsstoffe II und III sind in der Regel bekannt. Sofern sie nicht bekannt sind, können sie nach an sich bekannten Methoden hergestellt werden. So sind 7-Cyan-2-brom-3,3-dimethyl-1,2,3,4-tetrahydro-1-naphthol (II, $R^1$ = $R^2$ = $CH_3$, X-Y = -CHOH-CHBr-, $R^6$ = 6-CN, $R^7$ = H) und 7-Cyan-1,2-epoxy-3,3-dimethyl-1,2,3,4-tetrahydronaphthalin ("IIa"; = II, $R^1$ = $R^2$ = $CH_3$,

X-Y = 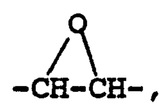,

$R^6$ = 6-CN, $R^7$ = H) in der EP-A-168619 beschrieben.

In Verbindungen der Formel II, worin -X-Y- -CHE-$CR^3R^8$-bedeutet, kommen als "reaktionsfähig veresterte OH-Gruppen" insbesondere Alkylsulfonyloxy mit 1-6 C-Atomen (z.B. Methansulfonyloxy) und Arylsulfonyloxy mit 6-10 C-Atomen (z.B. Benzolsulfonyloxy, p-Toluolsulfonyloxy, 1- oder 2-Naphthalinsulfonyloxy) in Betracht.

Als reaktionsfähige Derivate von III eignen sich die entsprechenden Salze, z. B. die Na- oder K-Salze, die auch in situ entstehen können.

Bei der Reaktion von II mit III ist es zweckmäßig, in Gegenwart einer Base zu arbeiten. Als Basen eignen sich z. B. Alkalimetall- oder Erdalkalimetall-hydroxide, -carbonate, -alkoholate, -hydride oder auch -amide wie NaOH, KOH, $Ca(OH)_2$, $Na_2CO_3$, $K_2CO_3$, Na- oder K-methylat, -ethylat oder -tert.-butylat, NaH, KH, $CaH_2$, $NaNH_2$, $KNH_2$, ferner organische Basen wie Triethylamin oder Pyridin, die auch im Überschuß angewendet werden und dann gleichzeitig als Lösungsmittel dienen können.

Als inerte Lösungmittel eignen sich insbesondere Alkohole wie Methanol, Ethanol, Isopropanol, n-Butanol oder tert.-Butanol; Ether wie Diethylether, Diisopropylether, Tetrahydrofuran oder Dioxan; Glykolether wie Ethylenglykolmonomethyl- oder -monoethylether (methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Arbeitet man mit NaH in DMSO bei Raumtemperatur, so erhält man ganz überwiegend die bevorzugten Verbindungen der Formel I, in denen $R^3$ und $R^4$ zusammen eine Bindung und Z eine Bindung bedeuten; so entstehen z.B. mit 1H-2-Pyridon die entsprechenden 1,2-Dihydro-4-(2-oxo-1,2-dihydro-pyridyl)-naphthaline.

Das Epoxid II

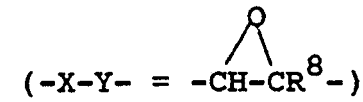

kann auch in situ hergestellt werden, z. B. durch Einwirkung einer Base auf das entsprechende Bromhydrin.

Eine Verbindung der Formel I, worin $R^3$ = OH und $R^4$ = H ist, kann durch Behandeln mit einem Dehydratisierungsmittel in eine Verbindung der Formel I, worin $R^3$ und $R^4$ zusammen eine Bindung bedeuten, umgewandelt werden. Das gelingt z. B. durch Einwirkung einer der angegebenen Basen, z. B. NaH, in einem der angegebenen Lösungsmittel, z. B. DMSO, bei Temperaturen zwischen 0 und 150 °.

Weiterhin kann man in einer Verbindung der Formel I einen oder mehrere der Reste $R^3$, $R^5$, $R^6$ und/oder $R^7$ in andere Reste $R^3$, $R^5$, $R^6$ und/oder $R^7$ umwandeln.

Beispielsweise ist es möglich, daß man ein H-Atom mittels einer Halogenierung durch ein Halogenatom oder mittels einer Nitrierung durch eine Nitrogruppe ersetzt und/oder eine Nitrogruppe zu einer Aminogruppe reduziert und/oder eine Amino- oder Hydroxygruppe alkyliert oder acyliert und/oder eine Cyangruppe (z.B. mit HCl in Wasser/Methanol bei 20-100°) in eine Carboxylgruppe oder (z. B. mit Raney-Nickel in Wasser/Essigsäure/Pyridin in Gegenwart von Natriumphosphat) in eine Formylgruppe oder (z. B. mit KOH in tert.-Butanol) in eine Carbamoylgruppe oder (z. B. mit $H_2S$ in Pyridin/Triethylamin) in eine Thiocarbamoylgruppe umwandelt und/oder eine -CO-NH-Gruppe (z. B. mit $P_2S_5$ oder mit Lawesson-Reagenz in Toluol) in eine -CS-NH- bzw. eine -C(SH)=N-Gruppe umwandelt.

Eine Nitrierung gelingt unter üblichen Bedingungen, z. B mit einem Gemisch aus konzentrierter $HNO_3$ und konzentrierter $H_2SO_4$ bei Temperaturen zwischen 0 und 30 °. Falls mindestens einer der Substituenten $R^6$ und $R^7$ eine elektronegative Gruppe wie CN oder $NO_2$ bedeutet, erfolgt die Nitrierung überwiegend am Rest $R^5$; andernfalls erhält man in der Regel Gemische, bei denen die Nitrogruppen am Rest $R^5$ oder am Benzolring stehen können.

Analoges gilt für die Halogenierung, die z. B. mit elementarem Chlor oder Brom in einem der üblichen inerten Lösungsmittel bei Temperaturen zwischen etwa 0 und 30 ° durchgeführt werden kann.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z. B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z. B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungsmittel, z. B. DMF, bei Temperaturen zwischen etwa 0 ° und etwa 120 ° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z. B. Chloride oder Bromide) oder Anhydride von Carbonsäuren der Formel Ac-OH, z. B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z. B. eines Kohlenwasserstoffs wie Toluol, eines Nitrils wie Acetonitril, eines Amids wie DMF oder eines Überschusses einer tertiären Base wie Pyridin oder Triethylamin bei Temperaturen zwischen etwa 0 ° und etwa 160 °, vorzugsweise zwischen 20 ° und 120 °. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure,

Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z. B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z. B. Verbindungen der Formel I, worin $R^1$ = $R^2$, $R^3$ = OH und $R^4$ = H ist, zwei chirale Zentren; bei der Herstellung durch Reaktion von II mit III entsteht jedoch ganz überwiegend nur ein Racemat mit trans-Stellung der Substituenten $R^3$ = OH und $R^5$-Z. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z. B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Carbinole (I, $R^3$ = OH) können ferner mit Hilfe chiraler Acylierungsreagenzien, z. B. D- oder L-α-Methylbenzylisocyanat, verestert und dann getrennt werden (vgl. EP-A1-120428). Die verschiedenen Formen der Diastereomeren können in an sich bekannter Weise, z. B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline, Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z. B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsulffizienz, Angina pectoris, Arrhythmie, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bzw. Blutdrucksenkern, z. B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Verbindungen der Formel I und ihre Salze eignen sich, insbesondere bei topischer Anwendung, ferner zur Behandlung der Alopezie einschließlich der androgenen Alopezie und der Alopecia areata. Hierfür werden insbesondere phar mazeutische Zubereitungen verwendet, die zur topischen Behandlung der Kopfhaut geeignet und die oben genannt sind. Sie enthalten etwa 0,005 bis 10, bevorzugt 0,5 bis 3 Gew.% mindestens einer Verbindung der Formel I und/oder mindestens eines ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in °C angegeben.

Beispiel 1

Zu einer Anschlämmung von 3,1 g NaH in 100 ml trockenem DMSO tropft man unter Einleiten von $N_2$ und Rühren eine Lösung von 9,2 g 1H-2-Pyridon in 100 ml DMSO. Nach 1 Std. Rühren tropft man eine Lösung von 15 g IIa in 125 ml DMSO hinzu und Rührt weitere 17 Std. bei 20°. Danach versetzt man mit gesättigter NaCl-Lösung, extrahiert mit Ethylacetat, wäscht mit Wasser, trocknet über Natriumsulfat, dampft ein und chromatographiert an Kieselgel (Dichlormethan/Ethylacetat 8:2). Man erhält 2,2-Dimethyl-4-(2-oxo-1,2-dihydro-1-pyridyl)-6-cyan-1,2-dihydronaphthalin ("A"), F. 112-112,5°.

Analog erhält man mit 2,4-Dihydroxypyridin (=4-Hydroxy-1H-2-pyridon) das 2,2-Dimethyl-4-(2-oxo-4-hydroxy-1,2-dihydro-1-pyridyl)-6-cyan-1,2-dihydronaphthalin, F. 222°.

Analog erhält man mit 3,6-Dihydroxypyridazin (= 3-Hydroxy-6-oxo-1,6-dihydro-pyridazin) das 2,2-Dimethyl-4-(3-hydroxy-6-oxo-1,6-dihydro-1-pyridazinyl)-6-cyan-1,2-dihydronaphthalin.

Analog erhält man:

2,2-Dimethyl-4-(2-thioxo-1,2-dihydro-1-pyridyl)-6-cyan-1,2-dihydronaphthalin
2,2-Dimethyl-4-(2-oxo-3-chlor-1,2-dihydro-1-pyridyl)-6-cyan-1,2-dihydronaphthalin
2,2-Dimethyl-4-(2-oxo-5-chlor-1,2-dihydro-1-pyridyl)-6-cyan-1,2-dihydronaphthalin
2,2-Dimethyl-4-(2-oxo-6-chlor-1,2-dihydro-1-pyridyl)-6-cyan-1,2-dihydronaphthalin
2,2-Dimethyl-4-(2-oxo-3-hydroxy-1,2-dihydro-1-pyridyl)-6-cyan-1,2-dihydronaphthalin
2,2-Dimethyl-4-(2-oxo-5-hydroxy-1,2-dihydro-1-pyridyl)-6-cyan-1,2-dihydronaphthalin
2,2-Dimethyl-4-(2-oxo-3-methoxy-1,2-dihydro-1-pyridyl)-6-cyan-1,2-dihydronaphthalin
2,2-Dimethyl-4-(2-oxo-3-acetoxy-1,2-dihydro-1-pyridyl)-6-cyan-1,2-dihydronaphthalin
2,2-Dimethyl-4-(2-oxo-3-nitro-1,2-dihydro-1-pyridyl)-6-cyan-1,2-dihydronaphthalin
2,2-Dimethyl-4-(2-oxo-5-nitro-1,2-dihydro-1-pyridyl)-6-cyan-1,2-dihydronaphthalin
2,2-Dimethyl-4-(2-oxo-3-amino-1,2-dihydro-1-pyridyl)-6-cyan-1,2-dihydronaphthalin
2,2-Dimethyl-4-(2-oxo-5-amino-1,2-dihydro-1-pyridyl)-6-cyan-1,2-dihydronaphthalin
2,2-Dimethyl-4-(2-oxo-3-acetamido-1,2-dihydro-1-pyridyl)-6-cyan-1,2-dihydronaphthalin
2,2-Dimethyl-4-(2-oxo-5-acetamido-1,2-dihydro-1-pyridyl)-6-cyan-1,2-dihydronaphthalin
2,2-Dimethyl-4-(2-oxo-3-carboxy-1,2-dihydro-1-pyridyl)-6-cyan-1,2-dihydronaphthalin
2,2-Dimethyl-4-(2-oxo-3,5-dichlor-1,2-dihydro-1-pyridyl)-6-cyan-1,2-dihydronaphthalin
2,2-Dimethyl-4-(2-oxo-3,5-dibrom-1,2-dihydro-1-pyridyl)-6-cyan-1,2-dihydronaphthalin
2,2-Dimethyl-4-(2-oxo-3-chlor-5-nitro-1,2-dihydro-1-pyridyl)-6-cyan-1,2-dihydronaphthalin
2,2-Dimethyl-4-(2-oxo-3-nitro-5-chlor-1,2-dihydro-1-pyridyl)-6-cyan-1,2-dihydronaphthalin
2,2-Dimethyl-4-(2-oxo-3-brom-5-nitro-1,2-dihydro-1-pyridyl)-6-cyan-1,2-dihydronaphthalin
2,2-Dimethyl-4-(2-oxo-3-nitro-5-brom-1,2-dihydro-1-pyridyl)-6-cyan-1,2-dihydronaphthalin
2,2-Dimethyl-4-(2-oxo-3,5-dinitro-1,2-dihydro-1-pyridyl)-6-cyan-1,2-dihydronaphthalin

2,2-Dimethyl-4-(2-oxo-3-chlor-5-amino-1,2-dihydro-1-pyridyl)-6-cyan-1,2-dihydronaphthalin
2,2-Dimethyl-4-(2-oxo-3-amino-5-chlor-1,2-dihydro-1-pyridyl)-6-cyan-1,2-dihydronaphthalin
2,2-Dimethyl-4-(2-oxo-3-brom-5-amino-1,2-dihydro-1-pyridyl)-6-cyan-1,2-dihydronaphthalin
2,2-Dimethyl-4-(2-oxo-3-amino-5-brom-1,2-dihydro-1-pyridyl)-6-cyan-1,2-dihydronaphthalin
2,2-Dimethyl-4-(2-oxo-3-chlor-5-acetamido-1,2-dihydro-1-pyridyl)-6-cyan-1,2-dihydronaphthalin
2,2-Dimethyl-4-(2-oxo-3-acetamido-5-chlor-1,2-dihydro-1-pyridyl)-6-cyan-1,2-dihydronaphthalin
2,2-Dimethyl-4-(2-oxo-3-brom-5-acetamido-1,2-dihydro-1-pyridyl)-6-cyan-1,2-dihydronaphthalin
2,2-Dimethyl-4-(2-oxo-3-acetamido-5-brom-1,2-dihydro-1-pyridyl)-6-cyan-1,2-dihydronaphthalin
2,2-Dimethyl-4-(2-oxo-1,2-dihydro-1-pyridyl)-6-acetyl-1,2-dihydronaphthalin
2,2-Dimethyl-4-(2-oxo-1,2-dihydro-1-pyridyl)-6-methoxycarbonyl-1,2-dihydronaphthalin
2,2-Dimethyl-4-(2-oxo-1,2-dihydro-1-pyridyl)-6-ethoxycarbonyl-1,2-dihydronaphthalin
2,2-Dimethyl-4-(2-oxo-1,2-dihydro-1-pyridyl)-6-fluor-1,2-dihydronaphthalin
2,2-Dimethyl-4-(2-oxo-1,2-dihydro-1-pyridyl)-6-chlor-1,2-dihydronaphthalin
2,2-Dimethyl-4-(2-oxo-1,2-dihydro-1-pyridyl)-6-brom-1,2-dihydronaphthalin
2,2-Dimethyl-4-(2-oxo-1,2-dihydro-1-pyridyl)-6-nitro-1,2-dihydronaphthalin
2,2-Dimethyl-4-(2-oxo-1,2-dihydro-1-pyridyl)-6-trifluormethyl-1,2-dihydronaphthalin
2,2-Dimethyl-4-(2-oxo-1,2-dihydro-1-pyridyl)-6-acetamido-1,2-dihydronaphthalin
2,2-Dimethyl-4-(2-oxo-1,2-dihydro-1-pyridyl)-7-cyan-1,2-dihydronaphthalin
2,2-Dimethyl-4-(2-oxo-1,2-dihydro-1-pyridyl)-6-acetamido-7-nitro-1,2-dihydronaphthalin
2-Methyl-4-(2-oxo-1,2-dihydro-1-pyridyl)-6-cyan-1,2-dihydronaphthalin
2-Methyl-2-ethyl-4-(2-oxo-1,2-dihydro-1-pyridyl)-6-cyan-1,2-dihydronaphthalin
2,2-Diethyl-4-(2-oxo-1,2-dihydro-1-pyridyl)-6-cyan-1,2-dihydronaphthalin
2,2-Tetramethylen-4-(2-oxo-1,2-dihydro-1-pyridyl)-6-cyan-1,2-dihydronaphthalin
2,2-Pentamethylen-4-(2-oxo-1,2-dihydro-1-pyridyl)-6-cyan-1,2-dihydronaphthalin
2,2,3-Tetramethyl-4-(2-oxo-1,2-dihydro-1-pyridyl)-6-cyan-1,2-dihydronaphthalin
2,2-Dimethyl-4-(6-oxo-1,6-dihydro-1-pyridazinyl)-6-cyan-1,2-dihydronaphthalin
2,2-Dimethyl-4-(3-ethoxycarbonyl-6-oxo-1,6-dihydro-1-pyridazinyl)-6-cyan-1,2-dihydronaphthalin
2,2-Dimethyl-4-(2-oxo-1,2-dihydro-1-pyrimidinyl)-6-cyan-1,2-dihydronaphthalin
2,2-Dimethyl-4-(2-oxo-4-hydroxy-1,2-dihydro-1-pyrimidinyl)-6-cyan-1,2-dihydronaphthalin
2,2-Dimethyl-4-(6-oxo-1,6-dihydro-1-pyrimidinyl)-6-cyan-1,2-dihydronaphthalin
2,2-Dimethyl-4-(4-hydroxy-6-oxo-1,6-dihydro-1-pyrimidinyl)-6-cyan-1,2-dihydronaphthalin
2,2-Dimethyl-4-(2-oxo-1,2-dihydro-1-pyrimidinyl)-6-cyan-1,2-dihydronaphthalin
2,2-Dimethyl-4-(2-oxo-2,5-dihydro-1-pyrrolyl)-6-cyan-1,2-dihydronaphthalin
2,2-Dimethyl-4-(2-oxo-4-hydroxy-1,2-dihydro-1-pyridyl)-6-brom-1,2-dihydronaphthalin
2,2-Dimethyl-4-(2-oxo-4-hydroxy-1,2-dihydro-1-pyridyl)-6-nitro-1,2-dihydronaphthalin
2,2-Dimethyl-4-(2-oxo-4-hydroxy-1,2-dihydro-1-pyridyl)-6-methoxycarbonyl-1,2-dihydronaphthalin
2,2-Dimethyl-4-(3-hydroxy-6-oxo-1,6-dihydro-1-pyridazinyl)-6-brom-1,2-dihydronaphthalin
2,2-Dimethyl-4-(3-hydroxy-6-oxo-1,6-dihydro-1-pyridazinyl)-6-nitro-1,2-dihydronaphthalin
2,2-Dimethyl-4-(3-hydroxy-6-oxo-1,6-dihydro-1-pyridazinyl)-6-methoxycarbonyl-1,2-dihydronaphthalin

Beispiel 2

Ein Gemisch von 19,9 g IIa, 9,5 g 1H-2-Pyridon, 5 ml Pyridin und 50 ml Ethanol wird 2 Std. auf 100° (im Rohr) erhitzt. Man dampft ein, arbeitet wie üblich auf und erhält bei der chromatographischen Trennung "A" (F. 112-112,5°) neben 2,2-Dimethyl-4-(2-oxo-1,2-dihydro-1-pyridyl)-6-cyan-1,2,3,4-tetrahydro-3-naphthol ("B"; F. 201-203°) und 2,2-Dimethyl-4-(2-pyridyloxy)-6-cyan-1,2,3,4-tetrahydro-3-naphthol (F. 113-114°).

Analog erhält man die in Beispiel 1 angegebenen Verbindungen sowie die entsprechenden 3,4-Dihydro-3-hydroxy-verbindungen, z. B.

2,2-Dimethyl-4-(2-oxo-4-hydroxy-1,2-dihydro-1-pyridyl)-6-cyan-1,2,3,4-tetrahydro-3-naphthol
2,2-Dimethyl-4-(3-hydroxy-6-oxo-1,6-dihydro-1-pyridazinyl)-6-cyan-1,2,3,4-tetrahydro-3-naphthol
2,2-Dimethyl-4-(2-oxo-1,2-dihydro-1-pyridyl)-6-nitro-1,2,3,4-tetrahydro-3-naphthol
sowie entsprechenden Verbindungen mit Z = O, z. B.
2,2-Dimethyl-4-(2-oxo-1,2-dihydro-4-pyridyloxy)-6-cyan-1,2,3,4-tetrahydro-3-naphthol, F. 265-269°
2,2-Dimethyl-4-(6-oxo-1,6-dihydro-3-pyridazinyloxy)-6-cyan-1,2,3,4-tetrahydo-3-naphthol
2,2-Dimethyl-4-(2-oxo-1,2-dihydro-4-pyridyloxy)-6-nitro-1,2,3,4-tetrahydro-3-naphthol.

Beispiel 3

Eine Lösung von 3,18 g 2,2-Dimethyl-4,6-dibrom-1,2,3,4-tetrahydronaphthalin (erhältlich aus 2,2-Dimethyl-6-brom-1,2,3,4-tetrahydro-4-naphthol und $SOBr_2$) und 0,95 g 2H-1-Pyridon in 20 ml DMSO wird mit 1,2 g 80%igem NaH versetzt und 3 Tage bei 20° gerührt. Nach üblicher Aufarbeitung erhält man 2,2-Dimethyl-4-(2-oxo-1,2-dihydro-1-pyridyl)-6-brom-1,2,3,4-tetrahydronaphthalin.

Beispiel 4

Analog Beispiel 2 erhält man aus IIa und 2-Amino-3-hydroxypyridin das 2,2-Dimethyl-4-(3-hydroxy-2-pyridyl-amino)-6-cyan-1,2,3,4-tetrahydro-3-naphthol.

Beispiel 5

Analog Beispiel 2 erhält man aus IIa und 2-Mercaptopyridin das 2,2-Dimethyl-4-(2-pyridyl-thio)-6-cyan-1,2,3,4-tetrahydro-3-naphthol.

Beispiel 6

Analog Beispiel 1 erhält man aus IIa und 2-Mercaptopyridin das 2,2-Dimethyl-4-(2-pyridyl-thio)-6-cyan-1,2-dihydronaphthalin.

Beispiel 7

Ein Gemisch von 1 g 2,2-Dimethyl-4-(2-pyridyl-thio)-6-cyan-1,2,3,4-tetrahydro-3-naphthol, 0,3 g NaOH und 35 ml Dioxan wird 20 Min. gekocht. Man kühlt ab, filtriert, dampft das Filtrat ein und erhält nach üblicher Aufarbeitung 2,2-Dimethyl-4-(2-pyridyl-thio)-6-cyan-1,2-dihydronaphthalin.

Beispiel 8

Ein Gemisch von 2 g "B", 11,7 ml Ameisensäure und 3,3 ml Acetanhydrid wird 16 Std. bei 20° stehengelassen und anschließend 2 Std. auf 40° erwärmt. Nach Eindampfen und üblicher Aufarbeitung erhält man 2,2-Dimethyl-3-formyloxy-4-(2-oxo-1,2-dihydro-1-pyridyl)-6-cyan-1,2,3,4-tetrahydronaphthalin.

Beispiel 9

Ein Gemisch von 1 g "B" und 5 ml Acetanhydrid wird 1 Std. gekocht. Man kühlt ab, arbeitet wie üblich auf und erhält 2,2-Dimethyl-3-acetoxy-4-(2-oxo-1,2-dihydro-1-pyridyl)-6-cyan-1,2,3,4-tetrahydronaphthalin.

Beispiel 10

Ein Lösung von 1 g 2,2-Dimethyl-4-(2-oxo-1,2-dihydro-1-pyridyl)-6-nitro-1,2,3,4-tetrahydro-3-naphthol in 24 ml Methanol wird bei 20° und 1 bar an 0,5 g 5%igem Pd-C bis zum Stillstand hydriert. Man filtriert, dampft ein und erhält nach üblicher Aufarbeitung (mit verdünnter Natronlauge/Dichlormethan) 2,2-Dimethyl-4-(2-oxo-1,2-dihydro-1-pyridyl)-6-amino-1,2,3,4-tetrahydro-3-naphthol.

Beispiel 11

Ein Lösung von 1 g 2,2-Dimethyl-4-(2-oxo-1,2-dihydro-1-pyridyl)-6-amino-1,2,3,4-tetrahydro-3-naphthol in 15 ml Ameisensäure und 1 ml Pyridin wird 16 Std. gekocht und eingedampft. Nach üblicher Aufarbeitung

erhält man 2,2-Dimethyl-4-(2-oxo-1,2-dihydro-1-pyridyl)-6-formamido-1,2,3,4-tetrahydro-3-naphthol.

Beispiel 12

Ein Gemisch von 1 g 2,2-Dimethyl-4-(2-oxo-1,2-dihydro-1-pyridyl)-6-amino-1,2,3,4-tetrahydro-3-naphthol, 10 ml Acetanhydrid und 10 ml Pyridin wird 16 Std. bei 20° stehengelassen. Man dampft ein, reinigt chromatographisch und erhält 2,2-Dimethyl-4-(2-oxo-1,2-dihydro-1-pyridyl)-6-acetamido-1,2,3,4-tetrahydro-3-naphthol.

Beispiel 13

In eine siedende Lösung von 1 g "A" in 50 ml Methanol und 2 ml Wasser wird 14 Std. unter Rühren HCl eingeleitet. Man kühlt über Nacht auf 0°. Die ausgefallene 2,2-Dimethyl-4-(2-oxo-1,2-dihydro-1-pyridyl)-1,2-dihydronaphthalin-6-carbonsäure wird abfiltriert.

Beispiel 14

Ein Gemisch von 2,76 g "A", 31 g $Na_3PO_4 \cdot 12 H_2O$, 28 ml Pyridin, 28 ml Wasser, 67 ml Essigsäure und 25 g Raney-Nickel (wasserfeucht) wird 3 Std. bei 20° gerührt. Nach Filtration arbeitet man wie üblich auf und erhält 2,2-Dimethyl-4-(2-oxo-1,2-dihydro-1-pyridyl)-6-formyl-1,2-dihydronaphthalin.

Beispiel 15

Man löst 2,76 g "A" in 40 ml tert.-Butanol und versetzt unter Rühren mit 5,6 g gepulvertem KOH. Nach einstündigem Kochen und üblicher Aufarbeitung erhält man 2,2-Dimethyl-4-(2-oxo-1,2-dihydro-1-pyridyl)-6-carbamoyl-1,2-dihydronaphthalin.

Beispiel 16

In eine Lösung von 2,76 g "A" in einem Gemisch von 20 ml Pyridin und 10 ml Triethylamin leitet man bei 20° 5 Std. $H_2S$ ein, dampft ein, arbeitet wie üblich auf und erhält 2,2-Dimethyl-4-(2-oxo-1,2-dihydro-1-pyridyl)-6-thiocarbamoyl-1,2-dihydronaphthalin.

Beispiel 17

Ein Gemisch von 2,76 g "A", 8,08 g Lawesson-Reagenz und 50 ml Toluol wird 1 Std. unter $N_2$ gekocht. Übliche Aufarbeitung gibt 2,2-Dimethyl-4-(2-thioxo-1,2-dihydro-1-pyridyl)-6-cyan-1,2-dihydronaphthalin.

Analog erhält man

2,2-Dimethyl-4-(2-thioxo-1,2-dihydro-1-pyridyl)-6-brom-1,2-dihydronaphthalin

2,2-Dimethyl-4-(2-thioxo-1,2-dihydro-1-pyridyl)-6-nitro-1,2-dihydronaphthalin.

Beispiel 18

Ein Gemisch von 310 mg 2,2-Dimethyl-4-(2-oxo-1,2-dihydro-4-pyridyloxy)-6-cyan-1,2,3,4-tetrahydro-3-naphthol, 20 ml Aceton, 400 mg $K_2CO_3$ und 0,2 ml Dimethylsulfat wird 2 Std. gekocht. Man filtriert, arbeitet wie üblich auf und erhält 2,2-Dimethyl-4-(1-methyl-2-oxo-1,2-dihydro-4-pyridyloxy)-6-cyan-1,2,3,4-tetrahydro-3-naphthol.

Beispiel 19

Ein Gemisch von 311 mg 2,2-Dimethyl-4-(6-oxo-1,6-dihydro-3-pyridazinyloxy)-6-cyan-1,2,3,4-tetrahydro-3-naphthol, 1 g $K_2CO_3$, 0,65 ml Dimethylsulfat und 16 ml DMF wird 3 Std. gekocht und wie üblich aufgearbeitet. Man erhält 2,2-Dimethyl-4-(6-methoxy-3-pyridazinyloxy)-6-cyan-1,2,3,4-tetrahydro-3-naphthol.

Beispiel 20

Analog Beispiel 2 erhält man aus 1,2-Epoxy-1,2,3,4-tetrahydronaphthalin und 1H-2-Pyridon das 1-(2-Oxo-1,2-dihydro-1-pyridyl)-1,2,3,4-tetrahydro-2-naphthol, F. 180-181°. Daneben entsteht 1-(2-Oxo-1,2-dihydro-1-pyridyl)-3,4-dihydronaphthalin.

Analog erhält man mit 1,2-Epoxy-3,3-dimethyl-1,2,3,4-tetrahydronaphthalin das 1-(2-Oxo-1,2-dihydro-1-pyridyl)-3,3-dimethyl 1,2,3,4-tetrahydro-2-naphthol und das 1-(2-Oxo-1,2-dihydro-1-pyridyl)-3,3-dimethyl-3,4-dihydronaphthalin.

Analog erhält man mit 1,2-Epoxy-7-brom-1,2,3,4-tetrahydronaphthalin das 1-(2-Oxo-1,2-dihydro-1-pyridyl)-7-brom-1,2,3,4-tetrahydro-2-naphthol und das 1-(2-Oxo-1,2-dihydro-1-pyridyl)-7-brom-3,4-dihydronaphthalin.

Analog erhält man mit 1,2-Epoxy-7-cyan-1,2,3,4-tetrahydronaphthalin das 1-(2-Oxo-1,2-dihydro-1-pyridyl)-7-cyan-1,2,3,4-tetrahydro-2-naphthol und das 1-(2-Oxo-1,2-dihydro-1-pyridyl)-7-cyan-3,4-dihydronaphthalin.

Analog erhält man mit 3,4-Epoxy-6-brom-2,2-dimethyl-1,2,3,4-tetrahydronaphthalin das 2,2-Dimethyl-4-(2-oxo-1,2-dihydro-1-pyridyl)-6-brom-1,2,3,4-tetrahydro-3-naphthol und das 2,2-Dimethyl-4-(2-oxo-1,2-dihydro-1-pyridyl)-6-brom-1,2-dihydronaphthalin.

Analog erhält man mit 3,4-Epoxy-6-brom-2,2,3-trimethyl-1,2,3,4-tetrahydronaphthalin das 2,2,3-Trimethyl-4-(2-oxo-1,2-dihydro-1-pyridyl)-6-brom-1,2,3,4-tetrahydro-3-naphthol und das 2,2,3-Trimethyl-4-(2-oxo-1,2-dihydro-1-pyridyl)-6-brom-1,2-dihydronaphthalin.

Analog erhält man mit 3,4-Epoxy-6-cyan-2,2,3-trimethyl-1,2,3,4-tetrahydronaphthalin das 2,2,3-Trimethyl-4-(2-oxo-1,2-dihydro-1-pyridyl)-6-cyan-1,2,3,4-tetrahydro-3-naphthol und das 2,2,3-Trimethyl-4-(2-oxo-1,2-dihydro-1-pyridyl)-6-cyan-1,2-dihydronaphthalin.

Beispiel 21

Analog Beispiel 2 erhält man aus IIa und 3-Mercapto-6-hydroxy-pyridazin das 2,2-Dimethyl-4-(6-oxo-1,6-dihydro-3-pyridazinyl-thio)-6-cyan-1,2,3,4-tetrahydro-3-naphthol, F. 223-225°.

Beispiel 22

Eine Lösung von 1 g 2,2-Dimethyl-4-(6-oxo-1,6-dihydro-3-pyridazinyl-thio)-6-cyan-1,2,3,4-tetrahydro-3-naphthol in 10 ml Methanol wird bei 20° tropfenweise mit etherischer Diazomethanlösung bis zur Gelbfärbung versetzt. Man dampft ein und erhält 2,2-Dimethyl-4-(6-oxo-1-methyl-1,6-dihydro-3-pyridazinyl-thio)-6-cyan-1,2,3,4-tetrahydro-3-naphthol, F. 149-151°.

Die nachstehenden Beispiele betreffen pharmazeutische Zubereitungen, die Verbindungen der Formel I oder ihre physiologisch unbedenklichen Salze enthalten:

Beispiel A Tabletten

Ein Gemisch von 1 kg "A", 80 kg Lactose, 24 kg Kartoffelstärke, 4 kg Talk und 2 kg Magnesiumstearat wird in üblicher Weise zu Tabletten gepreßt, derart, daß jede Tablette 1 mg Wirkstoff enthält.

Beispiel B Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C Kapseln

Man füllt 1 kg 2,2-Dimethyl-4-(2-oxo-4-hydroxy-1,2-dihydro-1-pyridyl)-6-cyan-1,2-dihydronaphthalin in üblicher Weise in Hartgelatinekapseln, so daß jede Kapsel 0,5 mg Wirkstoff enthält.

Beispiel D Ampullen

Eine Lösung von 50 g "A" in 70 l 1,2-Propandiol wird mit zweifach destilliertem Wasser auf 100 l aufgefüllt steril filtriert, im Ampullen abgefüllt und steril verschlossen. Jede Ampulle enthält 0,5 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

## Ansprüche

1. Tetralinderivate der Formel I

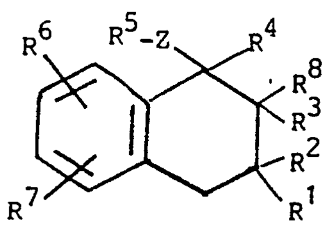

I

worin
$R^1$, $R^2$ und $R^8$ jeweils H oder A,
$R^1$ und $R^2$ zusammen auch Alkylen mit 3-6 C-Atomen,
$R^3$ H, OH, OA oder OAc,
$R^4$ H,
$R^3$ und $R^4$ zusammen auch eine Bindung,
$R^5$ einen unsubstituierten oder ein- oder zweifach durch A, F, Cl, Br, J, OH, OA, OAc, SH, $NO_2$, $NH_2$, AcNH, HOOC und/oder AOOC substituierten Pyridyl-, Pyridazinyl-, Pyrimi dinyl- Pyrazinyl-, Oxo-dihydro-pyridyl-, Oxo-dihydro-pyridazinyl-, Oxo-dihydro-pyrimidinyl-, Oxo-dihydro-pyrazinyl- oder Oxo-dihydro-pyrrolyl rest,
$R^6$ und $R^7$ jeweils H, A, HO, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitroalkyl, Cyanalkyl, A-C(=NOH) oder A-C(-$NNH_2$),
Z O, S, NH oder eine Bindung,
A Alkyl mit 1-6 C-Atomen,
-alkyl Alkylen mit 1-6 C-Atomen und
Ac Alkanoyl mit 1-8 C-Atomen oder Aroyl mit 7-11 C-Atomen
bedeuten,
sowie deren Salze.

2. a) 2,2-Dimethyl-4-(2-oxo-1,2-dihydro-1-pyridyl)-6-cyan-1,2-dihydro-naphthalin;
b) 2,2-Dimethyl-4-(2-oxo-4-hydroxy-1,2-dihydro-1-pyridyl)-6-cyan-1,2-dihydro-naphthalin;
c) 2,2-Dimethyl-4-(3-hydroxy-6-oxo-1,2-dihydro-1-pyridazinyl)-6-cyan-1,2-dihydro-naphthalin;

3. Verfahren zur Herstellung von Tetralinderivaten der Formel I gemäß Anspruch 1, dadurch gekennzeichnet, daß man ein Tetralin der Formel II

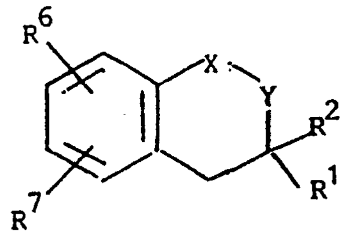

II

worin
X-Y

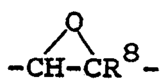

oder $-CHE-CR^3R^8-$ und
E Cl, Br, J oder eine reaktionsfähig veresterte OH-Gruppe bedeuten und
$R^1$, $R^2$, $R^3$, $R^6$, $R^7$ und $R^8$ die bei Formel I angegebenen Bedeutungen haben,
mit einer Verbindung der Formel III
$R^5$-ZH III
worin $R^5$ und Z die bei Formel I angegebenen Bedeutungen haben,
oder mit einem ihrer reaktionsfähigen Derivate umsetzt und/oder daß man eine Verbindung der Formel I, worin $R^3$ OH und $R^4$ H bedeutet, dehydratisiert und/oder daß man in einer Verbindung der Formel I einen oder mehrere der Reste $R^3$, $R^5$, $R^6$ und/oder $R^7$ in andere Reste $R^3$, $R^5$, $R^6$ und/oder $R^7$ umwandelt und/oder daß man eine basische Verbindung der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel zur Bekämpfung von Krankheiten.

7. Verwendung von Verbindungen der Formel I zur Herstellung eines Arzneimittels.

8. Verwendung von Verbindungen der Formel I bei der Bekämpfung von Krankheiten.

Europäisches Patentamt

# EUROPÄISCHER RECHERCHENBERICHT

Nummer der Anmeldung

EP 89 12 0367

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 168 619 (BEECHAM GROUP PLC)<br>* insgesamt *<br>--- | 1,5 | C 07 D 207/38<br>C 07 D 213/64<br>C 07 D 213/70<br>C 07 D 213/14<br>C 07 D 237/16<br>C 07 D 237/18<br>C 07 D 239/36<br>C 07 D 239/40<br>A 61 K 31/44 |
| Y | EP-A-0 273 262 (MERCK PATENT GMBH)<br>* Seiten 16-17; Ansprüche 1-3; Seite 8, Zeilen 17-22 *<br>--- | 1,5 | |
| Y | EP-A-0 218 373 (BEECHAM GROUP PLC)<br>* Seite 1, Anspruch 1; Seite 4, Anspruch 4; Seite 6, Anspruch 10 *<br>--- | 1,5 | |
| A | EP-A-0 089 781 (PFIZER INC.)<br>* Seiten 111-113; Beispiele 46-48 *<br>----- | 1 | |
| | | | **RECHERCHIERTE SACHGEBIETE (Int. Cl.5)** |
| | | | C 07 D 207/00<br>C 07 D 213/00<br>C 07 D 237/00<br>C 07 D 239/00 |

Der vorliegende Recherchenbericht wurde für alle Patentansprüche erstellt

| Recherchenort | Abschlußdatum der Recherche | Prüfer |
|---|---|---|
| BERLIN | 30-01-1990 | KYRIAKAKOU G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)